# EUROPÄISCHE PATENTSCHRIFT

(11) **EP 1 764 131 B1**
(45) Veröffentlichungstag und Bekanntmachung des Hinweises auf die Patenterteilung: **20.04.2011**
(21) Anmeldenummer: 06120101.8
(22) Anmeldetag: 05.09.2006
(51) Int. Cl.: A61N 5/00

(54) **Therapieanlage und Verfahren zur Zuordnung eines Partikelstrahls zu einem Bestrahlungsplatz**
Particle therapy device and method for allocating a particle beam to an irradiation site
Appareil et méthode de thérapie par particules pour l'allocation d'un faisceau de particules à un site d'irradiation

(30) Priorität: 16.09.2005 DE 102005044408; 16.09.2005 US 717834 P
(43) Veröffentlichungstag der Anmeldung: 21.03.2007
(73) Patentinhaber: SIEMENS AKTIENGESELLSCHAFT, 80333 München (DE)
(72) Erfinder: Birgy, Denis, 91091 Großenseebach (DE); Breuninger, Harald, 91330 Eggolsheim (DE)

(56) Entgegenhaltungen:
- EP-A- 1 454 657
- US-A- 5 260 581
- US-A1- 2005 029 472

## Beschreibung

Die Erfindung betrifft eine Partikeltherapieanlage mit einer Beschleuniger- und Partikelstrahlzuführungseinheit zur Beschleunigung von Partikeln und zur Zuführung von Partikeln an mindestens zwei Bestrahlungsplätzen und mit einer Zuordnungseinheit zur Sicherstellung und Überwachung der korrekten Partikelstrahlführung, wobei mindestens einer der Bestrahlungsplätze eine Kontrolleinheit aufweist.

Eine Partikeltherapieanlage weist üblicherweise eine Partikelbeschleunigereinheit, eine sich anschließenden Partikelstrahlzuführungseinheit sowie mehrere Bestrahlungsplätze auf. Die Beschleunigung der Partikel, z.B. Protonen, Pionen, Helium-, Kohlenstoff- oder Sauerstoff-Ionen, erfolgt beispielsweise mit Hilfe eines Synchrotrons oder Zyklotrons. Die beschleunigten hochenergetischen Partikel werden aus der Partikelbeschleunigereinheit ausgekoppelt und in die auch als Hochenergiestrahltransportsystem (high energy beam transport system HEBT) bezeichnete Partikelstrahlzuführungseinheit eingekoppelt. Im Falle eines Synchrotrons erfolgt die Auskopplung beispielsweise über einen Knock-Out-Exciter. Die HEBT erlaubt es, die hochenergetischen Partikel immer demjenigen Bestrahlungsplatz zuzuführen, an dem gerade ein Bestrahlungsvorgang erfolgen soll.

An einem im Folgenden auch als Behandlungsplatz bezeichneten Bestrahlungsplatz erfolgt z.B. eine Tumortherapie eines Patienten, der dazu im Partikelstrahlengang positioniert und den hochenergetischen Partikel ausgesetzt wird. Man unterscheidet zwischen "fixed beam" Behandlungsplätzen, in denen die Partikel aus einer festen Richtung auf einen Behandlungsplatz treffen, und so genannten Gantry basierten Behandlungsplätzen. Bei letzteren ist es möglich, den Partikelstrahl aus verschiedenen Richtungen auf den Behandlungsplatz der Gantry zu richten. Ferner erfolgt eine Überwachung der Strahlqualität an einem im Folgenden als Überprüfungsplatz bezeichneten Bestrahlungsplatz. Dort werden Strahlparameter wie Partikelenergie, Energieverteilung und Strahlintensität in Qualitätsmessungen überwacht.

An die Sicherheit einer Partikeltherapieanlage sind hohe Ansprüche gestellt. So muss z.B. gewährleistet sein, dass der Partikelstrahl immer nur zu einem Bestrahlungsplatz geführt wird, der auf einen Bestrahlungsvorgang vorbereitet ist und der den Partikelstrahl angefordert hat. Ferner muss gewährleistet sein, dass der Partikelstrahl die korrekten angeforderten Parameter aufweist. Ferner ist im Notfall eine schnelle Unterbrechung der Partikelzuführung notwendig. Dazu weist z.B. die HEBT eine Schikane auf, die es erlaubt den Partikelstrahl schnell abzuschalten. Üblicherweise gewährleistet ein Kontroll- und Sicherheitssystem der Partikeltherapieanlage, dass jeweils ein mit den benötigten Parametern charakterisierter Partikelstrahl in den entsprechenden Behandlungsraum geführt wird.

Die benötigten Parameter werden im so genannten Behandlungs- oder Therapieplan definiert. Dieser gibt an, wie viele Teilchen aus welcher Richtung mit welcher Energie auf den Patienten treffen sollen. Die Energie der Partikel bestimmt die Eindringtiefe der Partikel in den Patienten, d.h. den Ort, an dem das Maximum der Wechselwirkung mit dem Gewebe bei der Partikeltherapie erfolgt; in anderen Worten, den Ort, an dem das Maximum der Dosis deponiert wird. Die vom Therapieplan geforderten Parameter werden üblicherweise von einer Beschleunigerkontrolleinheit in Einstellparameter, z.B. in Form von Maschinenparametern, für die Beschleuniger- und Partikelstrahlzuführungseinheit umgesetzt. Ferner wird die Information, an welchen Bestrahlungsplatz der Partikelstrahl geführt werden soll, in Einstellparameter für die Partikelstrahlzuführungseinheit umgesetzt. Des Weiteren steuert eine Kontrolleinheit des Bestrahlungsplatzes beispielsweise eine Positioniervorrichtung, mit der ein zu bestrahlender Patient/ein zu bestrahlendes Phantom in Bezug zum Partikelstrahl positioniert wird.

Eine Partikeltherapieanlage mit mehreren Fixed-Beam-Behandlungsplätzen und einer Gantry ist z.B. aus EP 0 986 070 bekannt. Verschiedene Bestrahlungsanlagen und -techniken sind von H. Blattmann in "Beam delivery systems for charged particles", Radiat. Environ. Biophys. (1992) 31:219-231 beschrieben.

Ein Verfahren zur Auswahl eines Behandlungsraumes ist z.B. aus US 5,260,581 und EP 1 454 657 A2 bekannt. Ein Kontroll- und Sicherheitssystem für eine Strahlentherapieanlage ist z.B. aus US 5,895,926 bekannt.

Der Erfindung liegt die Aufgabe zugrunde, einen sicheren Betrieb einer Partikeltherapieanlage und insbesondere eine sichere Zuführung eines Partikelstrahls an einen den Partikelstrahl anfordernden Bestrahlungsplatz zu gewährleisten.

Diese Aufgabe wird erfindungsgemäß gelöst durch eine Partikeltherapieanlage nach Anspruch 1 sowie ein Verfahren und eine Vorrichtung zur Einstellung eines Strahlpfades nach Anspruch 11 bzw. 17. Ferner wird die Aufgabe gelöst durch die Verwendung einer direkten und fest zugeordneten Signalverbindung von einem von mehreren Bestrahlungsplätzen einer Partikeltherapieanlage mit einer Zuordnungseinheit der Partikeltherapieanlage zur Übermittlung eines Strahlanforderungssignals oder eines Bestätigungssignals.

Eine direkte und fest zugeordnete Signalverbindung ist z.B. eine direkte Hardware-Verbindung, insbesondere eine einzelne und bevorzugt eine sicherheitsgerichtete, Signalleitung. Dabei schließt direkt auch ein Zusammenklemmen von mehreren Kabelabschnitten ein, wobei ein einziges durchgehend verlegtes Kabel bevorzugt ist.

Eine direkte und fest zugeordnete Signalverbindung hat den Vorteil, dass eine eindeutige Zuordnung eines Bestrahlungsplatzes zu einem Signaleingang besteht. Dadurch ist es gewährleistet, dass immer der richtige Bestrahlungsplatz von der Zuordnungseinheit erkannt wird, ohne dass hier eine Bestätigung zur Verifikation des richtigen Bestrahlungsplatzes über z.B. ein Protokoll benötigt wird. Es besteht also eine Sicherheit bei der Zuordnung des Bestrahlungsplatzes ohne zusätzlichen Verifikationsschritt. Somit erlaubt eine auf diese Weise Hardware kodierte und kontrollierte Vorgehensweise ein eine gesicherte Zuführung eines Partikelstrahls entlang eines Partikelpfads zum anfordernden Bestrahlungsplatz. Die Signalverbindung wird vorzugsweise nur in eine Richtung verwendet, so dass eine störanfällige Logik zur Differenzierung der Richtung der Signalübertragung nicht benötigt wird.

Das Beruhen des Sicherheitskonzepts auf einer Hardware-Kodierung ist ein Vorteil gegenüber dem Verfahren in der eingangs genannten Schrift US 5,895,926. Denn eine gesicherte Zuordnung und/oder eine gesicherte Strahlverfügbarkeitskontrolle durch dedizierte Hardware-Signalleitungen erschwert Manipulationsmöglichkeiten gegenüber einer reinen Bus-Lösung, denen eine variable Signalgebung aufprägbar ist.

Eine Ausführungsform einer erfindungsgemäßen Partikeltherapieanlage weist beispielsweise eine Beschleuniger- und Partikelstrahlzuführungseinheit zur Beschleunigung von Partikeln und zur Zuführung von Partikeln vom Beschleuniger an mindestens zwei Bestrahlungsplätze auf. Eine derartige Einheit umfasst beispielsweise als Beschleuniger ein Zyklotron oder eine Synchrotron, in das evtl. vorbeschleunigte Partikel eingekoppelt werden. Die Partikelstrahlzuführung erfolgt z.B. mit Hilfe von mindestens einem einstellbaren Element im Strahlpfad. Das oder die Elemente werden mit Hilfe der Beschleunigerkontrolleinheit entsprechend dem jeweils benötigten Strahlpfad eingestellt. Zur Einstellung benötigte Einstellparameter werden übermittelt und z.B. in einem Zwischenspeicher abgelegt.

Ferner umfasst die Partikeltherapieanlage eine Zuordnungseinheit zur Sicherstellung und Überwachung einer korrekten Partikelstrahlführung entlang des Strahlpfades. Dieser verläuft innerhalb der Beschleuniger- und Partikelstrahlzuführungseinheit zu einem den Partikelstrahl anfordernden Bestrahlungsplatz. Die Zuordnungseinheit ist beispielsweise eine sicherheitsgerichtete und speicherprogrammierbare Steuerungseinheit.

Mindestens einer der Behandlungsplätze weist eine Kontrolleinheit auf, die über einer direkte und fest zugeordnete Signalverbindung direkt mit einem Signaleingang der Zuordnungseinheit verbunden ist und die die zur Abgabe eines Anforderungssignals zur Anforderung eines Partikelstrahls für einen Bestrahlungsvorgang über die Signalverbindung ausgebildet ist, so dass ein Anliegen des Anforderungssignals am Signaleingang eindeutig den anfordernden Bestrahlungsplatz festlegt.

Vorzugsweise besteht zwischen der Zuordnungseinheit und der Kontrolleinheit des Bestrahlungsplatzes eine zweite fest zugeordnete Signalverbindung zur Übermittlung eines Bestätigungssignals von einem Signalausgang der Zuordnungseinheit zur Kontrolleinheit.

Üblicherweise wird die Therapieanlage mehrere Bestrahlungsplätze aufweisen. Diese sind jeweils einzeln über fest zugeordnete Signalverbindung direkt mit je einem Signaleingang (und evtl. Signalausgang) der Zuordnungseinheit verbunden. Damit sind die Kontrolleinheiten über eine direkte Hardwareverbindung beispielsweise durch einzelne, direkte Signalleitungen mit der Zuordnungseinheit verbunden.

Üblicherweise wird die Beschleuniger- und Partikelstrahlzuführungseinheit mehrere Elemente aufweisen. Diese sind z.B. ebenfalls jeweils einzeln über je eine fest zugeordnete Signalverbindung direkt mit je einem Signalausgang der Zuordnungseinheit verbunden. Damit sind z.B. auch die einstellbaren Elemente über eine direkte Hardwareverbindung beispielsweise durch einzelne, direkte Signalleitungen mit der Zuordnungseinheit verbunden.

Beispiele für einstellbare Elemente sind Strahlumlenkmagnete, die den Partikelstrahl von dem Strahlzuführungssystem in die einzelnen Behandlungsräume ablenken, eine Strahlauskopplungsvorrichtung eines Beschleunigers, beispielsweise ein Knock-Out-Exciter eines Synchrotron-Rings, ein Dipolmagnet einer Schikane im HEBT. Mögliche Einstellparameter sind entsprechend das anliegende Magnetfeld, ein dafür benötigter einzustellender Stromwert oder eine HF-Auskoppelfrequenz. Ein einstellbares Element ist vorzugsweise zur Verarbeitung und in Abhängigkeit vom Vorliegen des Aktivierungssignals zur Umsetzung des mindestens einen übermittelten Einstellparameters ausgebildet. Dazu weist es beispielsweise einen Pufferspeicher auf, in den ein übermittelter Einstellparameter abgelegt und nach Erhalt des Aktivierungssignals ausgelesen werden kann.

Eine Übermittlung der Einstellparameter erfolgt beispielsweise über ein Datenbus-System, an das die Beschleunigerkontrolleinheit und die jeweiligen Element angebunden sind. Der Einstellparameter wird durch den am Bestrahlungsplatz stattfindenden Bestrahlungsvorgang bedingt.

Ebenfalls vorteilhaft ist es, wenn mindestens einer Kontrolleinheit der Bestrahlungsplätze zum Austausch von zur Bestrahlung benötigten Parametern des Partikelstrahls und/oder Parameter der Beschleuniger- und Partikelstrahlzuführungseinheit an dieses Datenbus-System oder an einem eigenen Datenbus-System angebunden sind.

Beispiele für Bestrahlungsplätze sind ein Behandlungsplatz zur Strahlentherapie, z.B. ein Fixed-Beam- oder Gantry-Behandlungsplatz, oder ein Überprüfungsplatz zur Überprüfung von der Partikelbestrahlung zugrunde liegenden Parametern.

Gemäß dem Verfahren zur Anforderung eines Partikelstrahls werden wird ein Anforderungssignal von einem der Bestrahlungsplätze an die Zuordnungseinheit über eine direkte und feste zugeordnete Signalverbindung gesendet. Dann wird ein Strahlpfad in der Partikeltherapieanlage eingestellt, der Partikel zum anfordernden Bestrahlungsplatz führt.

Weitere mögliche Vorgänge bei der Anforderung eines Strahls an einen Bestrahlungsplatz können folgende Schritte sein:
- Eine Verfügbarkeit des Partikelstrahls wird überprüft und der Partikelstrahl wird erst bei einer vorliegenden Verfügbarkeit des Partikelstrahls dem anfordernden Bestrahlungsplatz zugeteilt.
- Nach dem Zuteilen wird ein Bestätigungssignal an den anfordernden Bestrahlungsplatz, beispielsweise ebenfalls über eine direkte und feste zugeordnete Signalverbindung oder über ein Datenbussystem, geschickt.
- Nach Erhalt eines Bestätigungssignals wird vom anfordernden Bestrahlungsplatz aus der Partikelstrahl freigegeben ("Strahl an"-Signal), d.h., Partikel werden zum Behandlungsplatz geleitet.
- Ferner kann bei Eintreten eines Fehlers die Partikelbeschleunigung und/oder einer Weiterleitung der Partikel beispielsweise von der Zuordnungseinheit und/oder der Kontrolleinheit unterbrochen werden. Dazu wird, falls beispielsweise für ein Einstellen des Elements fortwährend ein Aktivierungssignal vorliegen muss, dieses Aktivierungssignal beendet. Wirkt das Aktivierungssignal als Schalter, kann des Element auf "nicht-einstellbar" geschaltet werden.

Weitere vorteilhafte Ausführungsformen der Erfindung sind durch die Merkmale der Unteransprüche gekennzeichnet.

Es folgt die Erläuterung von mehreren Gesichtspunkten der Erfindung anhand der Figuren 1 bis 3. Es zeigen:
Figur 1 einen schematischen Überblick über eine Therapieanlage,
Figur 2 eine schematische sicherheitsgerichtete Schalteinheit und
Figur 3 eine Skizze zur Anordnung von sicherheitsgerichteten Verbindungen.

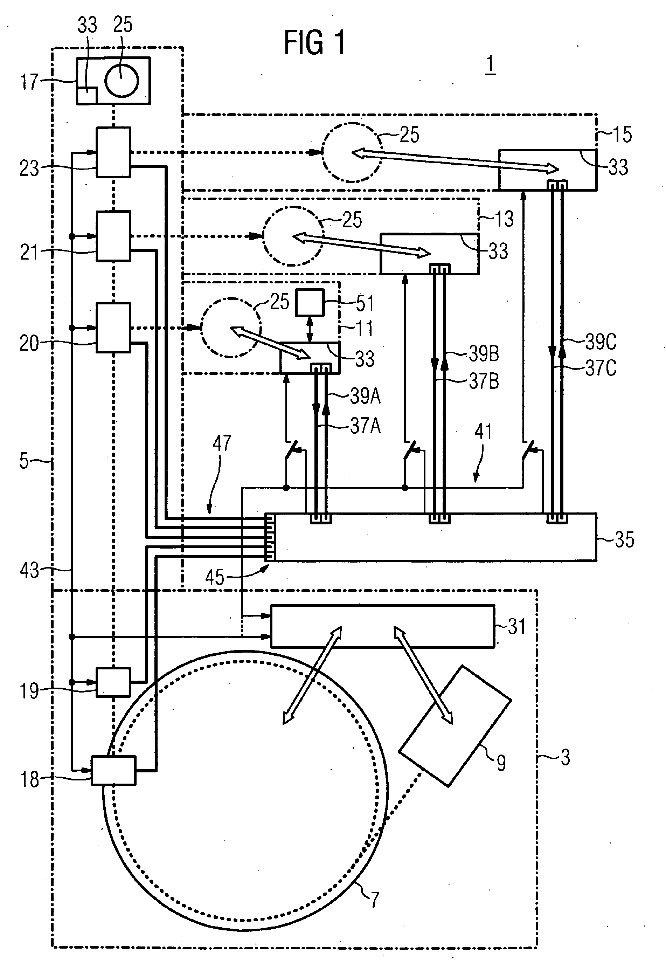

Figur 1 zeigt schematisch eine Therapieanlage 1 und verdeutlicht das Zusammenspiel von verschiedenen involvierten Kontrolleinheiten. Diese bewirkten und überwachen die Einstellung von Komponenten, um einen Strahl mit entsprechenden Parametern an einen Bestrahlungsplatz zu senden. Aus Sicherheitsgründen werden dabei wichtige Signale über Fehler unanfällige Hardware-Verbindung übermittelt. Die Hardware-Verbindung besteht z.B. aus einer eigenen, separaten spezifischen Leitung und ist eindeutig der Übertragung eines Signals zugeordnet.

Die Therapieanlage 1 weist eine Beschleunigereinheit 3 und eine Partikelstrahlzuführungseinheit 5 auf. Beispielhaft für einen Beschleuniger wurde ein Synchrotron 7 mit einer vorgeschaltenen Linearbeschleunigereinheit 9 dargestellt. Die Strahlzuführungseinheit 5 verteilt die Partikel auf mehrere Bestrahlungsplätze. Beispielhaft sind schematisch drei Behandlungsplätze 11, 13 und 15 zur Strahlentherapie und ein Überprüfungsplatz 17 zur Gewährleistung der Qualität des Partikelstrahls angedeutet. Am Überprüfungsplatz 17 erfolgt beispielsweise die Qualitätssicherung mit Hilfe von Qualitätsprozeduren, beispielsweise mit regelmäßigen Tests zur Verifikation der zuvor definierten Strahlparameter, beispielsweise von Positions- und Intensitätsabstufungen, von Partikelenergien. Letztere sind beispielsweise in einer Bibliothek enthalten und werden durch automatisierte Bragg-Peak-Messungen an Phantomen überprüft.

Mithilfe einer Auskoppelvorrichtung 18 werden im beispielsweise Synchrotronring 7 gespeicherte Partikel ausgekoppelt und in die Strahlzuführungseinheit 5 eingekoppelt. Die Möglichkeit einer schnellen Strahlabschaltung nach Beendigung oder Unterbrechung des Bestrahlungsvorgangs erfolgt beispielsweise durch den Einbau einer Schikane 19, welche beispielsweise aus drei kleinen Dipol-Magneten besteht, die nach der Extraktionseinheit 18 angeordnet sind. Durch eine schnelle Abschaltung des beispielsweise mittleren Dipols wird der Strahl an einem Kollimator vernichtet.

Die Zuführung der Partikel zu den Bestrahlungsplätzen 11, 13 und 15 erfolgt durch die Ablenkung des Partikelstrahls mittels Umlenkmagnete 20, 21 und 23 aus einer Hauptstrahlrichtung in der Strahlzuführungseinheit 5. In Hauptstrahlrichtung befindet sich der Überprüfungsplatz 17. An den Bestrahlungsplätzen erfolgt die Wechselwirkung der Partikel mit einem zu bestrahlenden Patienten oder einem Phantom in Bestrahlungszonen 25. Eine der Bestrahlungszonen 25 ist z.B. durch einen maximal abscanbaren Scanbereich einer (Raster-)Scanvorrichtung, einen maximal bestrahlbaren Scatterbereich einer Scattervorrichtung oder einen einstellbaren Gantry-Bestrahlungsbereich etc. gegeben.

Die Linearbeschleunigereinheit 9 kann beispielsweise ein oder mehrere betreibbare Ionenquellen, eine Niedrigenergiestrahlführung, einen Radio-Frequenz-Quadrupol, einen Drift-Röhrenbeschleuniger und eine Injektionsstrahlführung aufweisen. Aufgaben der Injektoreinheit 9 sind die Erzeugung einer oder mehrerer Teilchensorten, deren Befreiung von Verunreinigungen durch nicht gewünschte Teilchensorten, die Einstellung der Strahlintensität im Niedrigenergiebereich beispielsweise für das Synchrotron, eine Vorbeschleunigung der Partikel und eine Vorbereitung des Partikelstrahls beispielsweise in der Pulslänge und den Strahlparametern entsprechend den Anforderungen des Synchrotrons.

Wird die Therapieanlage 1 zur Bestrahlung mittels Scanning-Verfahren eingesetzt, ist eine langsame Extraktion von Vorteil, die eine optimale Nutzung der beschleunigten Teilchen und eine präzise Strahlüberwachung während der Tumorabtastung ermöglicht. In diesem Fall ist bei Verwendung eines Synchrotrons z.B. eine Strahlextraktion mit einer sog. HF-Knock-Out-Methode von Vorteil, bei der ein Knock-Out-Exciter die Auskoppeleinheit 18 bildet.

Das Kontroll- und Sicherheitssystem der Therapieanlage 1 ist zur Verdeutlichung in mehrere Komponenten aufgeteilt. Eine Aufteilung kann auch anders oder gar nicht vorgenommen werden, solange die verschiedenen Aspekte bei der Überwachung berücksichtigt werden.

In der Ausführung nach Figur 1 sorgt eine Beschleunigerkontrolleinheit 31 dafür, dass der angeforderte Partikelstrahl entsprechend seiner Spezifikation im Behandlungsraum ankommt. An den Bestrahlungsplätzen angeordnete Kontrolleinheiten 33 steuern den Ablauf eines Bestrahlungsvorgangs und sorgen dafür, dass der Partikelstrahl entsprechend einer Bestrahlungsplanung auf einen Patienten trifft.

Ferner umfasst das Kontroll- und Sicherheitssystem eine Zuordnungseinheit 35. Diese gewährleistet es, dass nur derjenige Bestrahlungsplatz 11,...17 einen Partikelstrahl zugeführt bekommt, der ihn auch angefordert hat. Dazu ist die Zuordnungseinheit 35 einerseits mit den Kontrolleinheiten 33 zumindest zur Übermittlung eines Anforderungssignals über eine feste und eindeutig zugeordnete Signalleitung 37A, 37B, 37C verbunden. Ferner kann eine weitere fest zugeordnete Signalleitung 39A, 39B, 39C zwischen der Zuordnungseinheit 35 und den Kontrolleinheiten 33 bestehen. Diese kann beispielsweise zur Übermittlung eines Bestätigungssignals von der Zuordnungseinheit 35 zu demjenigen Bestrahlungsplatz, dem als nächstes der Partikelstrahl zugeführt werden soll, verwendet werden.

Vorzugsweise weist das Kontroll- und Sicherheitssystem mindestens ein Datenbus-System 41 auf, an das die Kontrolleinheiten 33 und die Beschleunigerkontrolleinheit 31 angebunden sind. Es dient beispielsweise der Übermittlung von Einstellparametern für die Beschleunigereinheit 3 und die Partikelstrahlzuführungseinheit 5 für eine nächste durchzuführende Bestrahlung. Vorzugsweise kann die Zuordnungseinheit 35 derart auf das Datenbus-System 41 einwirken, dass nur derjenige Bestrahlungsplatz 11,...17, der ein Bestätigungssignal bekommen hat, Parameter übermitteln kann.

An ein weiteres, eventuell auch mit dem Datenbusses 41 zusammengelegtes, Datenbussystem 43 (gestrichelte Verbindung), sind die Beschleunigerkontrolleinheit 31 und von diesem einstellbare Elementen der Beschleuniger- und Strahlzuführungseinheit angebunden. In der Ausführungsform gemäß Figur 1 sind dies beispielsweise die Auskoppeleinheit 18, die Schikane 19 und die Umlenkmagneten 20, 21, 23. Über das Datenbussystem 43 werden diejenigen Einstellparameter an die Elemente übermittelt, die diese Elemente zur Einstellung des aktuell angeforderten Partikelstrahlpfades und für den Transport der Partikel mit der richtigen Energie etc. benötigen. Ein Strahlpfad bedingt Einstellungen von Elemente im Hochenergiestrahlpfad in Abhängigkeit eines festgelegten Bestrahlungsplatzes.

Allerdings kann eine Umsetzung der Einstellparameter nur dann erfolgen, wenn zusätzlich ein Aktivierungssignal der Zuordnungseinheit 35 an dem jeweils einzustellenden Element vorliegt. Dazu sind die einstellbaren Elemente mit Signalausgängen 45 der Signalzuordnungseinheit 35 über direkte, fest zugeordnete Signalleitungen 47 verbunden.

Die Tatsache, dass Anforderungs- und/oder Aktivierungssignale über spezifische unzweideutige Hardware-Verbindungen gesendet und empfangen werden, reicht aus zu gewährleisten, dass das Anforderungssignal von einem gewissen und bekannten Bestrahlungsplatz gesendet wurde und/oder dass nur explizit aktivierte Elemente zur Festlegung des Strahlpfades eingestellt werden. Es ist nicht möglich, dass Signale fehlerhaft von anderen Bestrahlungsplätzen empfangen oder zu anderen Elementen übermittelt werden.

Im Folgenden wird ein sicherer Ablauf einer Bestrahlung eines Patienten beschrieben, wie er beispielsweise unter Verwendung einer Anlage nach Figur 1 erfolgen kann. In einem Therapieplan 51 wird die Bestrahlung mit all seinen erforderlichen Parametern wie Strahleinfallsrichtung, Strahlintensität, Partikelart, Partikelenergie etc. festgelegt.

Nachdem der Bestrahlungsplan für den Patienten am Bestrahlungsplatz geladen, alle sicherheitstechnischen Voraussetzungen erfüllt und der Patient entsprechend positioniert wurde, fordert ein Therapiekontrollsystem beispielsweise eine Kontrolleinheit der Bestrahlungsplätze 11,... 15 einen Strahl mit den geplanten Parametern für den aktuellen Bestrahlungsplatz an. Vorzugsweise können nur getestete und freigegebene Datensätze von Parametern verwendet und angefordert werden, welche im Beschleunigerkontrollsystem 31 abgespeichert vorliegen.

Für die Strahlanforderung veranlasst ein Bediener die Sendung eines Anforderungssignals von beispielsweise der Kontrolleinheit des Bestrahlungsplatzes 11 entlang der direkten fest zugeordneten Signalleitung 37A an die Zuordnungseinheit 35. Die Zuordnungseinheit 35 überprüft die Verfügbarkeit des Partikelstrahls. Findet noch an einem Nachbarbestrahlungsplatz ein Bestrahlungsvorgang statt, teilt die Zuordnungseinheit erst mit Beendigung dieses Bestrahlungsvorgangs dem anfordernden Behandlungsraum den Partikelstrahl zu. Die Zuordnungseinheit gibt beispielsweise erst zu diesem Zeitpunkt die Verbindung von der Kontrolleinheit 33 des Behandlungsraums 11 zur Beschleunigerkontrolleinheit 31 im Datenbus-System 41 für die Übermittlung der gewünschten Parameter für den folgenden Bestrahlungsvorgang frei.

Zusätzlich sendet die Zuordnungseinheit 35 an die für die Strahlzuführung zum anfordernden Bestrahlungsplatz benötigten einstellbaren Elemente, in diesem Fall die Auskoppeleinheit 18, die Schikane 19 und der Umlenkmagnet 20, über die fest zugeordneten Signalleitungen 47 Aktivierungssignale. Ferner übermittelt die Beschleunigerkontrolleinheit 31 Einstellparameter in diese Elementen. Nur bei Vorlage des Aktivierungssignals können die von der Beschleunigerkontrolleinheit 31 übermittelten Einstellparameter in den Elementen umgesetzt werden und den benötigten Partikelstrahlpfad festlegen. Vorzugsweise sind die einstellbaren Elemente vorrangig deaktiviert. Nur bei Vorliegen eines Aktivierungssignals werden entsprechende Ströme etc. eingestellt. Deaktivierung bedeutet, dass beispielsweise der Defaultwert "Strom auf Null" eingestellt wird. D.h., zur Übermittlung eines Aktivierungssignals ist ein Signalausgang der Zuordnungseinheit über eine direkte und fest zugeordnete Signalverbindung mit mindestens einem der einstellbaren Elemente verbunden. Nur in Zusammenspiel mit dem Aktivierungssignal kann eine Umsetzung übermittelten Einstellparameters im Element erfolgen. D.h., das Aktivierungssignal muss z.B. vor und/oder während der Umsetzung vorliegen. Prinzipiell ist es vorzugsweise so vorgesehen, dass die einstellbaren Elemente vorrangig deaktiviert sind, d.h., die Beschleunigerkontrolleinheit wirkt als Verriegelungsmechanismus. Nur bei Vorliegen eines Aktivierungssignals werden entsprechende Ströme etc. eingestellt. Deaktivierung bedeutet, dass beispielsweise die Defaultwerte eingestellt sind, z.B. kein Stromfluss in den Magnetspulen.

Nach erfolgter Einstellung übermittelt die Zuordnungseinheit 35 entlang der direkten Verbindungsleitung 39A ein Bestätigungssignal. Nach einer evtl. Bestätigung dieses Signals durch den Behandlungsplatz 11 erfolgt die Zuführung von Partikeln zur Bestrahlung im Bestrahlungsbereich 25.

Die Reihenfolge von Einstellvorgängen und Signalübermittlungen ist -bis auf das Vorhandensein eines Aktivierungssignals für die tatsächliche Umsetzung von physikalischen Einstellungen- frei gestaltbar. So kann bei einem alternativen Ablauf beispielsweise direkt nach der Zuordnung des Partikelstrahls an den Behandlungsraum 11 das Bestätigungssignal an den Behandlungsraum 11 entlang der Verbindungsleitung 39A übermittelt werden. Ein aktiv von Seiten der Kontrolleinheit 33 des Behandlungsraums auf das Bestätigungssignal ausgelöstes "Strahl an"-Signal veranlasst Aktivierungssignale von der Zuordnungseinheit sowie die Übermittlung von Einstellparameter von der Beschleunigerkontrolleinheit 31 an die relevanten Elemente. Anschließend erfolgt die physikalische Umsetzung der Einstellparameter in den Elementen und die Partikel werden dem Bestrahlungsplatz zugeführt. Dieser Ablauf hat den Vorteil, dass die Umsetzung erst nach erfolgtem Bestätigungssignal vorgenommen wird und eine eventuelle Fehleinstellung somit frühzeitig verhindert werden kann.
Beispielsweise kann im Fall, dass eine nicht anfordernde Kontrolleinheit ein Bestätigungssignal erhält, automatisch ein entsprechende Deaktivierung vorgenommen werden.

Das zuletzt beschriebene Vorgehen lässt sich in drei Stufen untergliedern. In einer ersten Stufe zur Vorbereitung kommunizieren nur die Kontrolleinheit und die Zuordnungseinheit (Strahlanfragesignal, Bestätigungssignal der Strahlzuordnung sowie "Strahl an"-Signal. In einer zweiten Stufe der Einstellung kommunizieren die zugeordnete Kontrolleinheit und die Beschleunigerkontrolleinheit, d.h., es werden entsprechende Strahlparameter angefordert und die entsprechenden Parameter an die Elemente und die Beschleunigereinheit übermittelt. In einer dritten Stufe der Aktivierung kommuniziert die Zuordnungseinheit direkt mit den Elementen und macht die jeweils benötigten Elemente einstellbar, so dass die von der Beschleunigerkontrolleinheit übermittelten Parameter umgesetzt werden können. Die dritte Stufe macht die Einstellung physikalisch möglich und setzt sie um; sie kann auch schon zeitgleich mit der zweiten Stufe erfolgen.

Während des Bestrahlungsvorgangs arbeitet die Therapieanlage vollkommen autark. Z.B. steuert die Kontrolleinheit 33 Scannermagnete und Strahldiagnoseeinheiten zur Überwachung der Strahlqualität. Die einzige Eingriffsmöglichkeit für das Betriebspersonal ist ein Abbruch des Bestrahlungsvorgangs. Im Fall eines ausgelösten Strahlabbruchs oder eines anderweitig erkannten Fehlers im System entzieht die Zuordnungseinheit 35 über die direkten und fest zugeordneten Signalleitungen zu den einstellbaren Elementen die Erlaubnis, aktiviert zu sein. Bei Eintritt eines derartigen Fehlers mit erfolgt z.B. eine Strahlvernichtung innerhalb der Schikane durch Herunterfahren eines Dipolmagnetfeldes. Zusätzlich werden z.B. die Umlenkmagnete 20,21,23 stromlos geschaltet und der KO-Exciter ausgeschaltet.

Nach Abschluss eines Bestrahlungsvorganges können die einstellbaren Elemente wieder auf ihre Default-Werte eingestellt; so werden z.B. die Umlenk- und/oder Schikanenmagnetfelder auf Null gefahren sowie die KO-Frequenz ausgeschaltet. In Wechselwirkung mit einem die Ausnutzung optimierenden Betriebssystems der Therapieanlage zur Steuerung von anfallenden Bestrahlungsvorgängen kann ein Default-Wert evtl. in Hinblick auf den als nächstes erfolgenden Bestrahlungsvorgangs durch entsprechende Steuerung der Zuordnungseinheit übersprungen werden, so dass der Strahlenpfad schneller für den folgenden Bestrahlungsvorgang zur Verfügung steht.

Die Aufgaben der verschiedenen Komponenten des Kontroll- und Sicherheitssystems für die Strahlanforderung und Strahlpfadfestlegung lassen sich wie folgt zusammenfassen:
- Die Beschleunigerkontrolleinheit 31 kontrolliert die richtigen Werte der Einstellparameter für die einstellbaren Elemente in der Beschleuniger- und Strahlzuführungseinheit.
- Die Zuordnungseinheit 35 gewährt die Einstellbarkeit dieser Parameter mittels eines Aktivierungsprozesses, bei dem gezielt nur diejenigen Elemente aktiviert werden, die für einen Strahlpfad notwendig sind. Die Zuordnungseinheit hat hierfür vorzugsweise eine Tabelle mit den möglichen Strahlpfaden in beispielsweise einem Lock-up-Table gespeichert. Zusätzlich erfolgt eine Prüfung der Verfügbarkeit des Partikelstrahls innerhalb der bevorzugt als sicherheitsgerichtete speicherprogrammierbare Steuerung ausgebildeten Zuordnungseinheit 35. Eine Zuteilung des Partikelstrahls erfolgt nur bei dessen Verfügbarkeit.
- Die Kontrolleinheiten in den Bestrahlungsplätzen liefern die Daten aus dem Bestrahlungsplan und entscheiden letztendlich über die Zuführung des Strahls, d.h., sie lösen die Strahlzuführung an dem entsprechenden Bestrahlungsplatz aus.

Die Verwendung der direkten und fest zugeordneten Verbindungen ist nicht auf die in der Figur skizzierte Ausführungsform beschränkt. So kann beispielsweise nur die Verbindung zwischen einer der Kontrolleinheiten und der Zuordnungseinheit derart ausgeführt sein und die Einstellung von Elementen anders sichergestellt werden.

Diese Aufteilung ist beispielhaft zu sehen. Z.B. können Funktionen auch innerhalb einer Einheit zusammengefasst werden, beispielsweise können Parameter für den Therapieplan direkt und nicht über eine Kontrolleinheit eines Behandlungsraumes der Beschleunigerkontrolleinheit zugeführt werden.

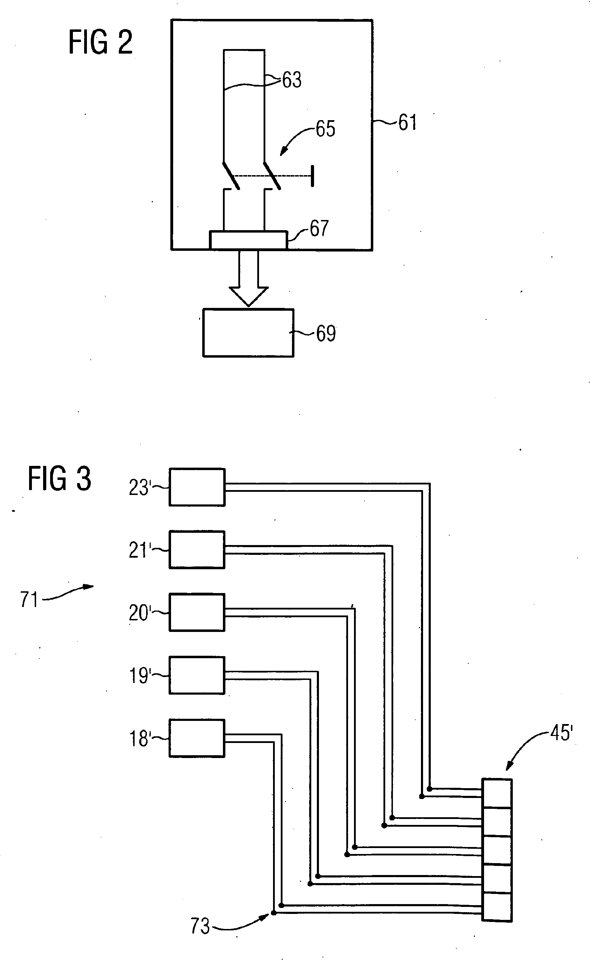

In den Figuren 2 und 3 werden Aspekte einer sicherheitsgerichteten direkten Verbindung verdeutlicht. Figur 2 zeigt schematisch eine sicherheitsgerichtete Schalteinheit 61, wie sie z.B. in der Kontrolleinheit und/oder der Zuordnungseinheit z.B. zur Übermittlung eines Anforderungs-, Bestätigungs- und/oder Aktivierungssignals verwendet werden kann. Zwei parallel geschaltete Leitungen 63 sind über einen zwangsöffnenden/zwangsschließenden Schalter 65 mit einem Signalausgang 67 verbindbar. Der Schalter 65 öffnet/schließt die beiden Leitungen 63 gemeinsam und nimmt im Fehlerfall einen sicheren Zustand ein. Der Signalausgang ist mit einer Einheit 69 verbunden; d.h. übertragen auf Figur 1 ist die Einheit 69 z.B. eine der Kontrolleinheiten 33, die Zuordnungseinheit 35 oder eines der aktivierbaren Elemente wie Auskoppeleinheit 18, Schikane 19 oder einer der Umlenkmagnete 20, 21, 23.

Figur 3 verdeutlicht den Einsatz von sicherheitsgerichteten Doppel-Leitungen für die Übermittlung von Aktivierungssignalen an einzustellenden Elemente 71 wie eine Auskoppeleinheit 18', eine Schikane 19' und Umlenkmagnete 20', 21', 23'. Diese werden von entsprechenden sicherheitsgerichtete Schalteinheit an Signalausgängen 45' angelegt, welche gemäß Figur 1 Teil der Zuordnungseinheit sind. Evtl. ist der Einsatz von Aufklemmungen 73 aufgrund der Größe einer Therapieanlage nicht vermeidbar.

## Patentansprüche

1. Partikeltherapieanlage (1) mit einer Beschleuniger- (3) und Partikelstrahlzuführungseinheit (5) zur Beschleunigung von Partikeln und zur Zuführung von Partikeln an mindestens zwei Bestrahlungsplätzen (11, 13, 15, 17) und mit einer Zuordnungseinheit (35) zur Sicherstellung und Überwachung der korrekten Partikelstrahlführung, wobei mindestens einer der Bestrahlungsplätze (11, 13, 15, 17) eine Kontrolleinheit (33) aufweist,
**dadurch gekennzeichnet,**
**dass** die Kontrolleinheit (33) über eine erste fest zugeordnete Signalverbindung (37A, 37B, 37C), die eine einzelne Signalleitung ist, direkt mit einem Signaleingang der Zuordnungseinheit (35) verbunden ist und dass die Kontrolleinheit (33) zur Abgabe eines Anforderungssignals zur Anforderung eines Partikelstrahls für einen Bestrahlungsvorgang über die Signalverbindung (37A, 37B, 37C) ausgebildet ist, so dass ein Anliegen des Anforderungssignals am Signaleingang eindeutig den anfordernden Bestrahlungsplatz festlegt.

2. Partikeltherapieanlage nach Anspruch 1,
**dadurch gekennzeichnet,**
**dass** zwischen der Zuordnungseinheit (35) und der Kontrolleinheit (33) des Bestrahlungsplatzes (11, 13, 15, 17) eine zweite fest zugeordnete Signalverbindung (39A, 39B, 39C) zur Übermittlung eines Bestätigungssignals von einem Signalausgang der Zuordnungseinheit (35) zur Kontrolleinheit (33) besteht.

3. Partikeltherapieanlage nach Anspruch 1 oder 2,
**dadurch gekennzeichnet,**
**dass** die zweite fest zugeordnete Signalverbindung (37A, 37B, 37C) eine direkte Hardware-Verbindung, insbesondere eine einzelne Signalleitung, ist.

4. Partikeltherapieanlage nach einem der Ansprüche 1 bis 3,
**dadurch gekennzeichnet,**
**dass** mindestens zwei Bestrahlungsplätze (11, 13, 15, 17) je mindestens eine Kontrolleinheit (33) aufweisen, die einzeln über eine fest zugeordnete Signalverbindung (37A, 37B, 37C) direkt mit einem Signaleingang der Zuordnungseinheit (35) verbunden sind.

5. Partikeltherapieanlage nach einem der Ansprüche 1 bis 4,
**dadurch gekennzeichnet,**
**dass** mindestens zwei Kontrolleinheiten (33) einzeln über je eine fest zugeordnete Signalverbindung (37A, 37B, 37C) direkt mit je mindestens einem Signalausgang der Zuordnungseinheit (35) verbunden sind.

6. Partikeltherapieanlage nach einem der Ansprüche 1 bis 5,
**dadurch gekennzeichnet,**
**dass** die Zuordnungseinheit (35) und die mindestens eine Kontrolleinheit (33) zur Übermittlung eines Bestätigungssignals an ein Datenbus-System (41) angebunden sind.

7. Partikeltherapieanlage nach einem der Ansprüche 1 bis 6,
**dadurch gekennzeichnet,**
**dass** eine Beschleunigerkontrolleinheit (31) und die mindestens eine Kontrolleinheit (33) zum Austausch von zur Bestrahlung benötigten Parametern des Partikelstrahls und/oder der Beschleuniger- (3) und Partikelstrahlzuführungseinheit (5), welche insbesondere vom Kontrollsystem aufgrund eines dem Bestrahlungsvorgang zugrunde liegenden Therapieplans (51) festgelegt sind, an einem Datenbus-System (41) angebunden sind.

8. Partikeltherapieanlage nach einem der Ansprüche 1 bis 7,
**dadurch gekennzeichnet,**
**dass** mindestens einer der Bestrahlungsplätze ein Behandlungsplatz (11, 13, 15) ist, an dem ein Patient mit den Partikeln bestrahlt wird.

9. Partikeltherapieanlage nach einem der Ansprüche 1 bis 8,
**dadurch gekennzeichnet,**
**dass** mindestens einer der Bestrahlungsplätze ein Überprüfungsplatz (17) zur Überprüfung von die Partikelbestrahlung charakterisierenden Parametern ist.

10. Partikeltherapieanlage nach einem der Ansprüche 1 bis 9,
**dadurch gekennzeichnet,**
**dass** die Zuordnungseinheit (35) eine sicherheitsgerichtete speicherprogrammierbare Steuerungseinheit aufweist.

11. Verfahren zur Anforderung eines Partikelstrahls für einen Bestrahlungsvorgang mit einer Partikeltherapieanlage (1) mit mindestens zwei Bestrahlungsplätzen (11, 13, 15, 17), mit folgenden Verfahrensmerkmalen:
- Senden eines Anforderungssignals vom ersten Bestrahlungsplatz (11, 13, 15, 17) zur Zuordnungseinheit (35),
- Einstellen eines Strahlpfades in der Partikeltherapieanlage zum anfordernden Bestrahlungsplatz (11, 13, 15, 17),
**dadurch gekennzeichnet,**
**dass** mindestens ein erster der Bestrahlungsplätze (11, 13, 15, 17) über eine erste direkte, fest zugeordnete Signalverbindung (37A, 37B, 37C), die eine einzelne Signalleitung ist, mit einer Zuordnungseinheit (35) der Partikeltherapieanlage (1) verbunden ist und dass das Anforderungssignal über die erste Signalverbindung (37A, 37B, 37C) vom ersten Bestrahlungsplatz (11, 13, 15, 17) zur Zuordnungseinheit (35) gesendet wird.

12. Verfahren nach Anspruch 11,
**dadurch gekennzeichnet, dass** vor dem Einstellen eines Strahlpfades eine Verfügbarkeit des Partikelstrahls, insbesondere innerhalb einer speicherprogram mierbaren Steuerungseinheit, sicherheitsgerichtet überprüft wird und dass bei einer Verfügbarkeit des Partikelstrahls dieser dem anfordernden Bestrahlungsplatz (11, 13, 15, 17) zugeteilt wird.

13. Verfahren nach Anspruch 12,
**dadurch gekennzeichnet, dass** nach dem Zuteilen ein Bestätigungssignal an den Bestrahlungsplatz (11, 13, 15, 17) über eine zweite direkte Signalverbindung (39A, 39B, 39C) oder über ein Datenbus-System (41), an das die Kontrolleinheit (33) und die Zuordnungseinheit (35) angebunden sind, geschickt wird.

14. Verfahren nach einem der Ansprüche 11 bis 13,
**dadurch gekennzeichnet, dass** nach Empfangen eines Bestätigungssignals an einem Bestrahlungsplatz (11, 13, 15, 17), der kein Anforderungssignal gesendet hat, die Partikelbeschleunigung und/oder einer Weiterleitung der Partikel, insbesondere von der Zuordnungseinheit (35) und/oder der Korrektureinheit gesteuert, unterbrochen wird.

## Claims

1. Particle therapy system (1) having an accelerator (3) and particle beam delivery unit (5) for accelerating particles and for delivering particles to at least two radiation treatment stations (11, 13, 15, 17) and having an assignment unit (35) for ensuring and monitoring correct particle beam delivery, wherein at least one of the radiation treatment stations (11, 13, 15, 17) has a control unit (33),
**characterised in that**
the control unit (33) is connected via a first permanently assigned signal link (37A, 37B, 37C) which has a separate signal lead directly to a signal input of the assignment unit (35) and **in that** the control unit (33) is embodied for issuing a request signal for requesting a particle beam for an irradiation operation via the signal link (37A, 37B, 37C) so that the presence of the request signal at the signal input uniquely identifies the requesting radiation treatment station.

2. Particle therapy system according to claim 1,
**characterised in that**
between the assignment unit (35) and the control unit (33) of the radiation treatment station (11, 13, 15, 17) there exists a second permanently assigned signal link (39A, 39B, 39C) for transmitting a confirmation signal from a signal output of the assignment unit (35) to the control unit (33).

3. Particle therapy system according to claim 1 or 2,
**characterised in that**
the second permanently assigned signal link (39A, 39B, 39C) is a direct hardware link, in particular a separate signal lead.

4. Particle therapy system according to one of claims 1 to 3,
**characterised in that**
at least two radiation treatment stations (11, 13, 15, 17) each have at least one control unit (33) that is connected separately via a permanently assigned signal link (37A, 37B, 37C) directly to a signal input of the assignment unit (35).

5. Particle therapy system according to one of claims 1 to 4,
**characterised in that**
at least two control units (33) are connected separately via one permanently assigned signal link (37A, 37B, 37C) in each case directly to at least one signal output of the assignment unit (35) in each case.

6. Particle therapy system according to one of claims 1 to 5,
**characterised in that**
the assignment unit (35) and the at least one control unit (33) are linked to a data bus system (41) for the purpose of transmitting a confirmation signal.

7. Particle therapy system according to one of claims 1 to 6,
**characterised in that**
an accelerator control unit (31) and the at least one control unit (33) are linked to a data bus system (41) for the purpose of exchanging required irradiation-characterising parameters of the particle beam and/or of the accelerator (3) and particle beam delivery unit (5), which parameters have been specified in particular by the control system on the basis of a therapy plan (51) on which the irradiation operation is based.

8. Particle therapy system according to one of claims 1 to 7,
**characterised in that**
at least one of the radiation treatment stations (11, 13, 15, 17) is a treatment station (11, 13, 15) at which a patient is irradiated with the particles.

9. Particle therapy system according to one of claims 1 to 8,
**characterised in that**
at least one of the radiation treatment stations is a checking station (17) for checking parameters characterising the particle irradiation operation.

10. Particle therapy system according to one of claims 1 to 9,
**characterised in that**
the assignment unit (35) has a safety-related programmable logic controller unit.

11. Method for requesting a particle beam for an irradiation operation comprising a particle therapy system (1) having at least two radiation treatment stations (11, 13, 15, 17), said method having the following features:
- Sending a request signal from the first radiation treatment station (11, 13, 15, 17) to the assignment unit (35),
- Setting a beam path in the particle therapy system to the requesting radiation treatment station (11, 13, 15, 17),
**characterised in that**
at least a first of the radiation treatment stations (11, 13, 15, 17) is connected via a first direct, permanently assigned signal link (37A, 37B, 37C) which is a separate signal lead to an assignment unit (35) of the particle therapy system (1) and **in that** the request signal is sent via the first signal link (37A, 37B, 37C) from the first radiation treatment station (11, 13, 15, 17) to the assignment unit (35).

12. Method according to claim 11,
**characterised in that**
before a beam path is set the availability of the particle beam is checked in terms of safety, in particular within a programmable logic controller unit, and **in that** if the particle beam is available it will be allocated to the requesting radiation treatment station (11, 13, 15, 17).

13. Method according to claim 12,
**characterised in that**
after the particle beam has been allocated, a confirmation signal is sent to the radiation treatment station (11, 13, 15, 17) via a second direct signal link (39A, 39B, 39C) or via a data bus system (41) to which the control unit (33) and the assignment unit (35) are linked.

14. Method according to one of claims 11 to 13,
**characterised in that** if a confirmation signal is received at a radiation treatment station (11, 13, 15, 17) that has not sent a request signal, the particle acceleration and/or forwarding of the particles will be interrupted, in particular under the control of the assignment unit (35) and/or the correction unit.

## Revendications

1. Appareil de thérapie par particules (1) comprenant une unité d'accélération (3) et de livraison d'un faisceau de particules (5) pour accélérer des particules et délivrer des particules à au moins deux stations d'irradiation (11, 13, 15, 17) et comprenant une unité d'allocation (35) pour garantir et surveiller la bonne livraison du faisceau de particules, au moins une des stations d'irradiation (11, 13, 15, 17) étant pourvue d'une unité de contrôle (33),
**caractérisé en ce que**
l'unité de contrôle (33) est directement reliée à une entrée de signal de l'unité d'allocation (35) par le biais d'une première liaison de signal à affectation fixe (37A, 37B, 37C) qui est une ligne de signal unique, et que l'unité de contrôle (33) est exécutée pour délivrer, par le biais de liaison de signal (37A, 37B, 37C), un signal de demande pour demander un faisceau de particules pour un processus d'irradiation, de manière à ce qu'une application du signal de demande à l'entrée de signal détermine de façon univoque la station d'irradiation qui demande.

2. Appareil de thérapie par particules selon la revendication 1, **caractérisé en ce qu'**il existe, entre l'unité d'allocation (35) et l'unité de contrôle (33) de la station d'irradiation (11, 13, 15, 17), une seconde liaison de signal à affectation fixe (39A, 39B, 39C) pour la transmission d'un signal de confirmation d'une sortie de signal de l'unité d'allocation (35) vers l'unité de contrôle (33).

3. Appareil de thérapie par particules selon la revendication 1 ou 2, **caractérisé en ce que** la seconde liaison de signal à affectation fixe (37A, 37B, 37C) est une liaison matérielle directe, notamment une liaison de signal unique.

4. Appareil de thérapie par particules selon l'une des revendications 1 à 3, **caractérisé en ce qu'**au moins deux stations d'irradiation (11, 13, 15, 17) sont pourvues à chaque fois d'au moins une unité de contrôle (33), lesquelles unités sont reliées chacune directement à une entrée de signal de l'unité d'allocation (35) par le biais d'une liaison de signal à affectation fixe (37A, 37B, 37C).

5. Appareil de thérapie par particules selon l'une des revendications 1 à 4, **caractérisé en ce qu'**au moins deux unités de contrôle (33) sont reliées chacune directement à au moins une sortie de signal de l'unité d'allocation (35) par le biais à chaque fois d'une liaison de signal à affectation fixe (37A, 37B, 37C).

6. Appareil de thérapie par particules selon l'une des revendications 1 à 5, **caractérisé en ce que** l'unité d'allocation (35) et la au moins une unité de contrôle (33) sont reliées à un système de bus de données (41) pour la transmission d'un signal de confirmation.

7. Appareil de thérapie par particules selon l'une des revendications 1 à 6, **caractérisé en ce qu'**une unité de contrôle d'accélérateur (31) et la au moins une unité de contrôle (33) sont reliées à un système de bus de données (41) pour échanger des paramètres du faisceau de particules et/ou de l'unité d'accélération (3) et de livraison de faisceau de particules (5) nécessaires à l'irradiation, qui sont fixés notamment par le système de contrôle sur la base d'un plan thérapeutique (51) qui sert de base au processus d'irradiation.

8. Appareil de thérapie par particules selon l'une des revendications 1 à 7, **caractérisé en ce qu'**au moins une des stations d'irradiation est une station de traitement (11, 13, 15), au niveau de laquelle un patient est irradié par les particules.

9. Appareil de thérapie par particules selon l'une des revendications 1 à 8, **caractérisé en ce qu'**au moins une des stations d'irradiation est une station de surveillance (17) pour surveiller des paramètres caractérisant l'irradiation par particules.

10. Appareil de thérapie par particules selon l'une des revendications 1 à 9, **caractérisé en ce que** l'unité d'allocation (35) est pourvue d'une unité de commande à mémoire programmable relative à la sécurité.

11. Procédé de demande d'un faisceau de particules pour un processus d'irradiation avec un appareil de thérapie par particules (1) comprenant au moins deux stations d'irradiation (11, 13, 15, 17), ayant les caractéristiques de procédé suivantes :
- émission d'un signal de demande de la première station d'irradiation (11, 13, 15, 17) vers l'unité d'allocation (35),
- mise au point d'un trajet de faisceau dans l'appareil de thérapie par particules jusqu'à la station d'irradiation (11, 13, 15, 17) qui fait la demande,
**caractérisé en ce que**
au moins une première des stations d'irradiation (11, 13, 15, 17) est reliée à une unité d'allocation (35) de l'appareil de thérapie par particules (1) par le biais d'une première liaison de signal à affectation fixe directe (37A, 37B, 37C) qui est une liaison de signal unique, et que le signal de demande est envoyé de la première station d'irradiation (11, 13, 15, 17) à l'unité d'allocation (35) par le biais de la première liaison de signal (37A, 37B, 37C).

12. Procédé selon la revendication 11,
**caractérisé en ce que**, avant la mise au point d'un trajet de faisceau, on procède, au titre de la sécurité, à la vérification d'une disponibilité du faisceau de particules, notamment à l'intérieur d'une unité de commande à mémoire programmable, et **en ce que**, en cas de disponibilité du faisceau de particules, celui-ci est alloué à la station d'irradiation (11, 13, 15, 17) qui fait la demande.

13. Procédé selon la revendication 12, **caractérisé en ce que**, après l'allocation, un signal de confirmation est envoyé à la station d'irradiation (11, 13, 15, 17) par le biais d'une seconde liaison de signal directe (39A, 39B, 39C) ou par le biais d'un système de bus de données (41) auquel l'unité de contrôle (33) et l'unité d'allocation (35) sont reliées.

14. Procédé selon l'une des revendications 11 à 13, **caractérisé en ce que**, après la réception d'un signal de confirmation au niveau d'une station d'irradiation (11, 13, 15, 17) qui n'a envoyé aucun signal de demande, l'accélération des particules et/ou une transmission des particules est interrompue, notamment par une commande de l'unité d'allocation (35) et/ou de l'unité de correction.
